# EUROPEAN PATENT APPLICATION

(11) **EP 0 837 069 A1**
(43) Date of publication of application: **22.04.1998**
(21) Application number: 97117551.8
(22) Date of filing: 10.10.1997
(51) Int. Cl.: C07H 3/06, C07H 15/203

(54) **Storage-stabilization method of acarbose**

(30) Priority: 21.10.1996 JP 297062/96; 19.06.1997 JP 177833/97
(71) Applicant: BAYER AG, 51368 Leverkusen (DE)
(72) Inventor: Suzuki, Hidekazu, Otsu-city, Shiga-pref, 520-21 (JP)

(57) **Abstract**

[Subject]

To prevent color change in acarbose or acarbose-containing preparations and improve their storage stability.

[Means for solving the problem]

A method for preserving acarbose or acarbose-containing preparations which is characterized by keeping acarbose or acarbose-containing preparations under low oxygen concentration conditions.

## Description

### Detailed Explanation of the Invention:

### Field of Technology

This invention relates to a method for preserving acarbose or acarbose-containing preparations.

### Background Technology

Acarbose is a substance which is isolated and refined from a liquid culture medium of an amino sugar-producing microorganism of genus actinoplanes, one of the species belonging to actinomycete, which has an action to repress the activities of α-amylase and α-glucosidase (sucrase, maltase, etc.) participating in digestion and absorption of carbohydrate in the intestinal tract of human, whereby to retard digestion and absorption of gluside and inhibit blood sugar level rise after a meal. Acarbose is hence widely used for oral treating medicines for diabetes (see: Japanese Patent Publication Nos. 18797/1984, 39340/1995 and 45537/1995; Patent No. 2,502,551, etc.)

Acarbose is a non-proprietary name of 0-4, 6-dideoxy-4-[[(1S,4R,5S,6S)-4,5,6-trihydroxy-3-(hydroxymethyl)-2-cyclohexen-1-yl]amino]-α-D-glucopyranosyl-(1→4)-O-α-D-glucopyranosyl-(1→4)-D-glucopyranose. It is present as a white to light yellow powder.

Acarbose is highly hygroscopic, and it is known that storage stability of acarbose or acarbose-containing preparations is largely affected by their water content. For example, Japanese Patent Publication No. 39340/1995 teaches: when water content of an acarbose-containing tablet exceeds about 6 percent by weight, its acarbose content decreases during storage at 60°C for 6 weeks, and the tablet is discolored. Furthermore Igaku to Yakugaku (medical science and pharmacy), Vol. 32, No. 3, pp. 597-601 (September, 1994) reports: after 4 weeks storage of acarbose-containing tablets at 20°C. 75% humidity and 30°C. 90% humidity, they showed weight increase, drop in hardness and changes in appearance (white tablets changed to light yellow to light brown colors), although their effective ingredient content remained unchanged.

It is heretofore held, therefore, acarbose or acarbose-containing preparations must be preserved under dry condition. For example, normally and widely adopted PTP wrapping with PVC (polyvinyl chloride) is unsuitable for a long-term storage of acarbose-containing tablets because they change in color (from white to light yellow) during the storage under the influence of moisture in air which permeates through the wrapping film. Thus, a means has been adopted to use CPP (polypropylene) exhibiting less moisture permeability as the material for the PTP wrapping, and to further pack the wrapped tablets in an aluminum-laminated film with hermetic sealing. Even with such strict moistureproof packing, however, the tablets still occasionally show slight color change and their commercial value is liable to be impaired. The reason for such color change has heretofore been not understood and an effective means for its prevention has been eagerly sought.

I had engaged in concentrative studies in search for a method which enables complete prevention of color change in acarbose or acarbose-containing preparations during their storage and stable retention of quality of acarbose over a prolonged period, to discover that, quite unexpectedly, the color change in acarbose or acarbose-containing preparations can be perfectly prevented when they are kept under moistureproof conditions and in an atmosphere substantially free of oxygen, and storage stability of acarbose is markedly improved. The present invention has thus been completed.

### Disclosure of the invention:

Accordingly, the present invention provides a method for preserving acarbose or acarbose-containing preparations, which is characterized by keeping acarbose or acarbose-containing preparations in an atmosphere containing substantially no oxygen.

Here the "atmosphere containing substantially no oxygen" refers to an atmosphere in which the oxygen concentration is not higher than 1%, preferably not higher than 0.5%, inter alia, not higher than 0.1%. Such an atmosphere can be formed by a number of methods. e.g., replacing air inside a substantially gas-impermeable and moistureproof container such as a metallic container, glass bottle, an aluminum-laminated film bag or the like, with a substantially oxygen-free inert gas, e.g., nitrogen gas, carbon dioxide gas or the like; creating a vacuum in said container; or substantially eliminating oxygen from the air inside the container.

The simplest method for substantially eliminating oxygen from the air inside a moistureproof container comprises placing a deoxidant in the container and seal the same. As deoxidants to be used for eliminating oxygen, any kind of heretofore known deoxidants of inorganic type (iron powder, sulfite, etc.) and organic type (catechol, ascorbic acid, unsaturated chain hydrocarbon polymer, etc.) can be used [cf. Kinoh Hoso Jitsuyo Jiten (handbook for practical functional packing), Kabushiki Kaisha Fuji Techno System, August 1, 1994], those which can absorb oxygen even under low humidity are preferred. Shape of the deoxidant may be any of powder, granules, blocks, sheet, etc. for example; or it may take a form of sheet or film of a thermoplastic resin in which a deoxidant composition is dispersed. The deoxidant may also be used as wrapped in an air-permeable material, in such a form as small bags wrapped in an air-permeable material or a sheet covered or laminated with an air-permeable material.

Such deoxidants have been commercialized and as examples of which a series of products manufactured by Mitsubishi Gas Chemical Industries and sold under a tradename of "Ageless®" may be named. In particular, autoreaction type products which act under low water content, such as Ageless S, Ageless Z, Ageless G, and the like can be conveniently used.

On the other hand, acarbose is hygroscopic as aforesaid, and changes with time as it absorbs moisture. Obviously, therefore, it is desirable that acarbose bulk and acarbose-containing preparations such as tablets, granules, powders, etc. are themselves substantially free or water (i.e., having a water content not more than about 6% by weight, in particular, not more than about 5% by weight). It is also important to maintain such acarbose and acarbose-containing preparations of low water content in an atmosphere of a low water content, e.g., a dry atmosphere having a relative humidity of not more than 20%, preferably not more than 10%.

Such a dry atmosphere can be formed within a used container by, for example, putting a desiccant in an above-described gas-impermeable, moistureproof container together with a deoxidant and sealing the container. Examples of useful desiccants include phosphorus pentoxide, calcium chloride, silica gel, calcium oxide etc., silica gel being preferred. Silica gel has been commercialized in such forms as granules, beads and tablets and any of them can be used.

It is also permissible to use a deoxidant and desiccant as a mixture which may be suitably in such forms as powders, granules, tablets, etc.

It is undesirable that the desiccant. deoxidant and their mixtures should come to direct contact with acarbose or acarbose-containing preparations. Normally, therefore, they are wrapped in an air-permeable packing material and then put into the packing containers of acarbose or acarbose-containing preparations.

As substantially gas-impermeable moistureproof containers to be used for preservation of acarbose or acarbose-containing preparations, those having an oxygen permeability of not more than 20 ml/m²·atm. 24 Hr., in particular, not more than 10 ml/m²·atm. 24 Hr. (25°C, 60% RH), are preferred. Furthermore, for preventing acarbose from absorbing moisture, the containers preferably have a moisture permeability of not more than 10 g/m²·24 Hr., in particular, not more than 5 g/m²·24 Hr. (40°C. 90% RH). As specific examples, molded containers and cans of a metal such as iron, aluminum, etc.; glass bottles with packing plugs; molded containers or bags made of polyvinylidene chloride, polyvinyl alcohol, nylon, etc.; and molded containers or bags made of various plastic films laminated with aluminum foil or vacuum-deposited with aluminum, aluminum oxide or a silicon compound can be named.

Thus, according to the present invention, a package of acarbose or an acarbose-containing preparation, which comprises acarbose or an acarbose-containing preparation, a means for deoxidation and a substantially gas-impermeable, moistureproof container to accommodate them as above-described, is provided.

Acarbose or an acarbose-containing preparation can be placed in the container as it is, or it may be first put in a sealed, moistureproof container like a PTP, and thereafter preserved in a substantially gas-impermeable container together with a deoxidant and also optionally with a desiccant as the occasion demands.

Hereinafter the present invention is explained still more specifically, referring to working examples.

### Examples:

### Example 1

In an atmosphere of room temperature and about 60% RH, twenty (20) tablets each containing 50 mg of acarbose (Glucobay® Tablet 50 mg: Bayer Yakuhin K.K.) were sealed up either (1) by themselves, (2) with a desiccant, (3) with a deoxidant or (4) with a desiccant and a deoxidant, in each about 60 ml-capacity brown glass container and kept at 40°C. After one week, two weeks, four weeks and twelve weeks from the initiation of the test, changes in color tone of the tablets were measured with a color-difference meter (Micro Color: Dr. Lange GmbH). As the desiccant, Silica Gel W 2010 (Yamani Yakuhin K.K.) was used, and as the deoxidant, Ageless Z-20 PK (Mitsubishi Gas Chemical Industries) was used.

The results were as indicated in Table 1 below.

**TABLE 1**

| | Color Difference (ΔE) from Tablet Before the Test | | | |
|---|---|---|---|---|
| | After 1 week | After 2 weeks | After 4 weeks | After 12 weeks |
| (1) Tablet alone | 1.3 | 1.9 | 3.0 | 5.9 |
| (2) With desiccant | 1.0 | 1.5 | 2.3 | 5.2 |
| (3) With deoxidant | 1.0 | 1.4 | 1.4 | 1.7 |
| (4) With desiccant and deoxidant | 0.8 | 1.1 | 1.1 | 1.5 |
| n=20 | | | | |

### Example 2

In an atmosphere of room temperature and about 60% RH, ten (10) tablets each containing 50 mg of acarbose (Glucobay Tablet 50 mg: Bayer Yakuhin K. K.) were sealed up as wrapped with an aluminum-laminated film (polyester 12 µm / polyethylene 13 µm / aluminum 9 µm / polyethylene 40 µm), either (1) as PTP (CPP), (2) as PTP and with a desiccant, or (3) as PTP and with a deoxidant, and kept at 60°C. After one week, two weeks and three weeks from the initiation of the test, changes in color tone of the tablets were measured with a color-difference meter (Micro Color: Dr. Lange GmbH). Calcium chloride (in sheet form, Kabushiki Kaisha ID) and Ageless Z-20 (Mitsubishi Gas Chemical Industries) were used as the desiccant and deoxidant, respectively.

The results are indicated in Table 2.

**TABLE 2**

| | Color Difference (ΔE) from Tablet Before the Test | | |
|---|---|---|---|
| | After 1 week | After 2 weeks | After 3 weeks |
| CPP + aluminum bag | 1.3 | 1.9 | 3.1 |
| CPP + desiccant + aluminum bag | 1.1 | 1.9 | 2.6 |
| CPP + deoxidant + aluminum bag | 0.7 | 1.1 | 1.1 |
| n=10 | | | |

### Example 3

In a low temperature booth of 20°C and 45% RH, a Drum Hoop Mixer (Ikeda Kikai Sangyo Kabushiki Kaisha) was charged with 350 g of acarbose, 770 g of crystalline cellulose, 3.5 g of light silicic anhydride and 760 g of corn starch which had been dried to reduce its water content to no higher than 6%. The content was mixed for 10 minutes. Then 7 g of magnesium stearate was added thereto, followed by further 5 minutes' mixing. The mixture was pressed with a rotary-type tabletting machine (Correct 19K, Kikusui Seisakujo) to make tablets of each 7 mm in diameter and 135 mg by weight, thus providing 10,000 tablets each containing 25 mg of acarbose.

In an atmosphere of room temperature and about 60% RH, twenty (20) each of the above-obtained 25 mg-tablets and commercial 50 mg-tablets (Bayer Yakuhin K. K.) were placed either (1) alone or (2) with a deoxidant, in an approximately 60 ml-capacity brown glass containers which were hermetically sealed and kept at 40°C. After one month, two months and three months from the initiation of the test, changes in color tone of the tablets were measured with a color-difference meter (Micro Color: Dr. Lange GmbH). As the deoxidant, Ageless SA-20 (Mitsubishi Gas Chemical Industries) was used.

The results are indicated in Table 3.

**TABLE 3**

| | | Color Difference (ΔE) from Tablet Before the Test | | |
|---|---|---|---|---|
| | | After 1 month | After 2 months | After 3 months |
| 25 mg Tablet | (1) Tablet alone | 0.5 | 1.0 | 1.6 |
| do. | (2) With deoxidant | 0.2 | 0.4 | 0.5 |
| 50 mg Tablet | (3) Tablet alone | 0.7 | 1.6 | 2.5 |
| do. | (4) With deoxidant | 0.4 | 0.5 | 0.5 |
| n=20 | | | | |

### Example 4

In a low temperature booth of 20°C and 45% RH, a Drum Hoop Mixer (Ikeda Kikai Sangyo Kabushiki Kaisha) was charged with 700 g of acarbose, 420 g of crystalline cellulose, 3.5 g of light silicic anhydride and 760 g of corn starch which had been dried to reduce its water content to no higher than 6%. The content was mixed for 10 minutes. Then 7 g of magnesium stearate was added thereto, followed by further 5 minutes' mixing. The mixture was pressed with a rotary-type tabletting machine (Correct 19K, Kikusui Seisakujo) to make tablets of each 7 mm in diameter and 135 mg by weight, thus providing 10,000 tablets each containing 50 mg of acarbose.

In an atmosphere of about 60% RH, twenty (20) each of the above-obtained 50 mg-tablets were placed in various containers as follows, which were hermetically sealed and kept at 40°C:
(1) a brown glass container of approximately 60 ml in capacity.
(2) a brown glass container of approximately 60 ml in capacity, whose inside air had been replaced by nitrogen.
(3) a vacuum aluminum-laminated film container
(4) an aluminum-laminated film container containing a deoxidant (SD-100: Nippon Soda Co.)
After one month and two months from the initiation of the test, changes in color tone of the tablets were measured with a color-difference meter (Micro Color: Dr. Lange GmbH).

The results are indicated in Table 4.

**TABLE 4**

| Atmosphere Inside the Container | Color Difference (ΔE) from Tablet Before the Test | |
|---|---|---|
| | After 1 month | After 2 months |
| (1) air | 1.7 | 2.9 |
| (2) nitrogen | 0.9 | 0.9 |
| (3) vacuum | 0.7 | 0.7 |
| (4) air + deoxidant | 0.6 | 0.5 |
| n=20 | | |

## Claims

1. A method for preserving acarbose or acarbose-containing preparations, which is characterized by keeping acarbose or acarbose-containing preparations in an atmosphere containing substantially no oxygen.

2. A method for preserving acarbose or acarbose-containing preparations, which is characterized by keeping acarbose or an acarbose-containing preparation in a hermetically sealed, moistureproof container from which oxygen has been substantially removed and which is substantially gas-impermeable.

3. A method for preserving acarbose or acarbose-containing preparations, which is characterized by preserving acarbose or an acarbose-containing preparation under hermetically sealed condition in a substantially gas-impermeable, moistureproof container, together with a deoxidant.

4. A method for preserving acarbose or acarbose-containing preparations, which is characterized by keeping acarbose or an acarbose-containing preparation in a hermetically sealed, moistureproof container, and further preserving the same in a substantially gas-impermeable container together with a deoxidant.

5. A method according to any of Claims 1 to 4, which is characterized in that the relative humidity of the atmosphere within said gas-impermeable container is not higher than 20% and the oxygen concentration in said atmosphere is not higher than 1%.

6. A package of acarbose or an acarbose-containing preparation, which comprises acarbose or an acarbose-containing preparation, a means for deoxidation and a substantially gas-impermeable, moistureproof, hermetically sealed container accomodating them.
